# EUROPÄISCHE PATENTSCHRIFT

(11) **EP 4 437 337 B1**
(45) Veröffentlichungstag und Bekanntmachung des Hinweises auf die Patenterteilung: **09.07.2025**
(21) Anmeldenummer: 22821494.6
(22) Anmeldetag: 22.11.2022
(51) Int. Cl.: G01N 33/38

(54) **ANLAGE MIT EINEM PROBENLAGER ZUR VORBEREITUNG EINES PROBEMATERIALS DER ZEMENT- ODER KALKINDUSTRIE AUF EINE KALORIMETRISCHE MESSUNG**
SYSTEM HAVING A SAMPLE STORE FOR PREPARING A SAMPLE MATERIAL IN THE CEMENT OR LIME INDUSTRY FOR CALORIMETRIC MEASUREMENT
SYSTÈME AVEC STOCKAGE D'ÉCHANTILLONS POUR PRÉPARATION D'UN ÉCHANTILLON DE MATÉRIAU DANS LE SECTEUR DU CIMENT OU DE LA CHAUX EN VUE D'UNE MESURE CALORIMÉTRIQUE

(30) Priorität: 24.11.2021 DE 102021213232; 24.11.2021 BE 202105915
(43) Veröffentlichungstag der Anmeldung: 02.10.2024
(73) Patentinhaber: thyssenkrupp Polysius GmbH, 59269 Beckum (DE); thyssenkrupp AG, 45143 Essen (DE)
(72) Erfinder: RÜTHER, Thomas, 48317 Drensteinfurt (DE); ENDERS, Michael, 48143 Münster (DE)
(74) Vertreter: thyssenkrupp Intellectual Property GmbH
(86) Internationale Anmeldenummer: PCT/EP2022/082884
(87) Internationale Veröffentlichungsnummer: WO 2023/094410

(56) Entgegenhaltungen:
- CN-A- 111 239 187
- DE-A1- 102014 018 489
- US-A1- 2020 150 005
- US-A1- 2020 166 417

## Beschreibung

Die Erfindung betrifft eine Anlage mit einem Probenlager zur Vorbereitung eines Probematerials der Zement- oder Kalkindustrie auf eine kalorimetrische Messung, sowie ein Verfahren zur Bestimmung der Reaktivität eines Probenmaterials.

Bei der Herstellung von Klinker Zement werden üblicherweise spektroskopische und diffraktometrische Analyseverfahren verwendet, um die Reaktivität von Klinker und Zement vorherzusagen. Der Einsatz alternativer Rohstoffe und Brennstoffe erschwert allerdings eine solche Vorhersage, da auch untergeordnete, in der Analytik nicht bestimmte Inhaltsstoffe eine Reaktivitätsänderung von Klinker und Zement bewirken. Wichtige Beispiele hierzu sind Fluor bei der Klinkermineralisation, Schwefeloxid aus Petcoke oder Phosphor bei Einsatz von Tiermehl. Darüber hinaus können auch bei bekannter Chemie und Mineralogie die Verweilzeit der Rohstoffe im Ofen und Prozessparameter wie die Ofendrehung oder die Flammenlänge auf die Reaktivität Einfluss nehmen. Das Verständnis solcher Zusammenhänge ist eingeschränkt, da bisher prozessnahe Messwerte zur Reaktivität von Klinker und Zement vielfach fehlen. Analytisch kann die Reaktivität von Klinker und Zement nur mit einem erheblichen Zeitverzug durch Druckfestigkeitsuntersuchungen in Mörtel oder in Beton bestimmt werden. Die Druckfestigkeitsbestimmung ist allerdings zu zeitaufwändig, um die Ergebnisse zur Prozesskontrolle nutzen zu können.

Aus der DE 10 2014 018 489 A1 ist ein Verfahren zur Analyse eines Zementwerkstoffes bekannt. Eine bekannte Möglichkeit der Prozessregelung unter Nutzung von Reaktivitätsdaten besteht in der isothermen Wärmeflusskalorimetrie. Dieses Verfahren nutzt die aus der Probe über die Zeit freigesetzte Wärme als Maß der Reaktivität. So kann anhand charakteristischer Kurven absolut und im Verlauf das Reaktionsverhalten, gemessen, charakterisiert und als Signal in die Prozessteuerung eingeführt werden.

Aus der US 2020 / 015 005 A1 ist eine Probenmühle bekannt.

Bei der Ermittlung der aus der Probe über die Zeit freigesetzte Wärme treten allerdings regelmäßig Messfehler auf, die eine Fehlbestimmung der Reaktivität und eine entsprechende Fehlsteuerung des Prozesses zur Folge haben.

Davon ausgehend ist es Aufgabe der vorliegenden Erfindung, eine Vorrichtung und ein Verfahren zur Ermittlung der isothermen Wärmeflusskalorimetrie bereitzustellen, die eine geringe Fehler Anfälligkeit aufweist.

Diese Aufgabe wird erfindungsgemäß durch eine Anlage zur Bestimmung der Reaktivität eines Probenmaterials mit den Merkmalen des unabhängigen Vorrichtungsanspruchs 1 und durch ein Verfahren zur Bestimmung der Reaktivität eines Probenmaterials mit den Merkmalen des unabhängigen Verfahrensanspruchs 12 gelöst. Vorteilhafte Weiterbildungen ergeben sich aus den abhängigen Ansprüchen.

Die Erfindung umfasst eine Anlage zur Bestimmung der Reaktivität eines Probenmaterial aufweisend:
eine Dosiereinrichtung zum Dosieren des Probenmaterials in einen Probenbehälter,
ein Probenlager zum Temperieren des Probematerials in dem Probenbehälter, wie vorangehend beschrieben,
eine Mischvorrichtung zur Aufnahme des Probenbehälters mit dem temperierten Probematerial und zum Mischen des Probenmaterials in dem Probenbehälter und ein Kalorimeter zur Ermittlung der Reaktivität des gemischten Probenmaterials.

Das Probenlager zur Vorbereitung eines Probematerials der Zement- oder Kalkindustrie auf eine kalorimetrische Messung umfasst:
einen Aufnahmekörper mit zumindest einem oder eine Mehrzahl von Lagerplätzen zur Aufnahme jeweils eines Probenbehälters mit einem Probematerial und eine Temperierungseinrichtung zum Kühlen oder Erwärmen der Lagerplätze des Aufnahmekörpers.

Das Probematerial ist beispielsweise ein Klinker oder ein hydraulisches Bindemittel mit unterschiedlichen Zusammensetzungen verschiedener Werkstoffkomponenten, wie beispielsweise Klinker, Sulfatträger oder Zumahlstoffe aufweisen. Zumahlstoffe sind beispielsweise Hüttensand, Flugasche, Puzzolan, Kalkstein oder kalzinierter Ton. Das Probematerial ist vorzugsweise gemahlen, insbesondere pulverförmig.

Bei einer kalorimetrischen Messung des Probematerials wird vorzugsweise die Reaktivität des Probematerials ermittelt. Vorzugsweise wird dazu die von dem Probematerial emittierte Wärmemenge pro Zeit nach Zugabe einer Anregerflüssigkeit ermittelt. Die kalorimetrische Messung erfolgt vorzugsweise isotherm.

Bei dem Aufnahmekörper handelt es sich beispielsweise um einen Vollblock aus einem Metall. Der Aufnahmekörper weist vorzugsweise eine Kastenform auf und ist beispielsweise innen hohl ausgestaltet. Der Aufnahmekörper ist optional einteilig ausgebildet. Die Lagerplätze dienen der Aufnahme jeweils eines Probenbehälters, wobei die Lagerplätze vorzugsweise derart ausgebildet sind, dass ein Probenbehälter vollständig oder teilweise in dem Lagerplatz aufgenommen wird. Jeder Lagerplatz ist vorzugsweise zur Aufnahme jeweils genau eines Probenbehälters ausgebildet. Bei dem Probenbehälter handelt es sich beispielsweise um eine zylinderförmige Ampulle, die vorzugsweise mit einem Deckel, insbesondere mittels Drehverschluss, verschließbar ist. Die Lagerplätze sind beispielsweise als im Wesentlichen vertikale Aussparungen, insbesondere Bohrungen von oben in den Aufnahmekörper. Die Lagerplätze sind beispielsweise zylinderförmig ausgebildet und weisen einen runden, insbesondere kreisförmigen konstanten Querschnitt auf. Die Tiefe der Lagerplätze entspricht vorzugsweise mindestens 5% der Höhe des darin aufzunehmenden Probenbehälters, vorzugsweise maximal 4 Mal der Höhe des Probenbehälters, insbesondere 0,9 - 2 Mal der Höhe des Probenbehälters, vorzugsweise insbesondere 0,5 Mal der Höhe des Probenbehälters. Die Lagerplätze sind vorzugsweise in einer horizontalen Ebene, insbesondere in Reihen nebeneinander angeordnet und vorzugsweise gleichmäßig zueinander beabstandet. Insbesondere beträgt die Anzahl der Lagerplätze 10 bis 100, vorzugsweise 20 bis 80, insbesondere 50. Es ist ebenfalls denkbar, dass eine Mehrzahl von Lagerplätzen in vertikaler Richtung nebeneinander gestapelt innerhalb des Aufnahmekörpers angeordnet ist.

Die Temperierungseinrichtung dient dem Temperieren, insbesondere Kühlen oder Erwärmen der Lagerplätze, vorzugsweise des Probematerials in den Probenbehältern, die in den Lagerplätzen gelagert sind. Die Temperatur der Probenbehälter entspricht nach einer bestimmten Verweilzeit des Probenbehälters in dem jeweiligen Lagerplatz vorzugsweise der Temperatur des Lagerplatzes. Dadurch wird eine optimale Temperierung der Probenbehälter vor dem Ermitteln der Reaktivität des Probenmaterials erreicht und somit die Messgenauigkeit erhöht.

Das Probenlager ist vorzugsweise Teil einer Anlage zur Bestimmung der Reaktivität eines Probenmaterials aus der Zement- oder Kalkindustrie. Es werden beispielsweise gleiche Produkte zu unterschiedlichen Probenahmezeiten beprobt und verglichen. Nach einer Erkenntnis der Erfinder ist es für eine verlässliche Bestimmung der Reaktivität, beispielsweise über den initialen Peak der emittierten Wärme, erforderlich, dass zu Beginn der Messung eine vernachlässigbar geringe Temperaturdifferenz zwischen dem Probematerial und der Innentemperatur des Messraums des Kalorimeters besteht. Es ist offensichtlich, dass eine von der Innentemperatur des Kalorimeters abweichende Temperatur des Probematerials zu einer Fehlbestimmung der Reaktivität des Probenmaterials führt, da auch diese zusätzliche Wärmemenge aus dem Temperaturunterschied zwischen dem Probenmaterial und dem Innenraum des isothermen Wärmeflusskalorimeters der Reaktionsenthalpie der Probe zugeschlagen wird. Die Erfinder sind zu der Erkenntnis gelangt, dass insbesondere für die Messung schneller, prozessnaher isothermer Wärmeflussmessungen Temperaturunterschiede zwischen dem Probenmaterial und der Innentemperatur des Wärmeflusskalorimeters schädlich für die Interpretation des Messeergebnisses sind.

Neben der eigentlichen Umgebungstemperatur können, gemäß einer Erkenntnis der Erfinder, insbesondere abweichende Temperaturen von Prozessproben eine für die Wärmeflusskalorimetrie ungünstige Wärmesignatur aufweisen. Beispiele hierfür sind Proben von Klinker kurz nach der Herstellung im Klinkerofen oder auch Zementproben, die noch eine über der Umgebung liegende Temperatur aus dem Mahlprozess aufweisen oder auch eine unzureichend genaue Einststellung der Umgebungstemperatur um das in der Regel automatisierte isotherme Wärmeflusskalorimeter. Die Erfinder sind daher zu der Erkenntnis gelangt, dass das für die isotherme Wärmeflusskalorimetrie vorgesehene Probenmaterial idealerweise noch vor dem Beginn der Messung thermisch auf die Innentemperatur des Wärmeflusskalorimeters eingestellt werden muss.

Eine verlässliche Ermittlung der Reaktivität bietet den Vorteil einer optimalen Prozessführung, wobei zu unterschiedlichen Probenahmezeiten beprobte Produkte aus der Zementvermahlung direkt verglichen werden und ein Regeleingriff, zum Beispiel durch eine Änderung der Produktfeinheit oder des Klinkeranteils vorgenommen werden können. Analog kann der Klinker durch Variation der Rohstoffe und Brennstoffe im Sinne einer Reaktivitätsbestimmung auf einen Zielwert gesteuert werden.

Gemäß einer ersten Ausführungsform weist die Temperierungseinrichtung einen Wärmeträgerkreislauf mit einer Heiz-/Kühleinrichtung auf. Insbesondere umfasst die Temperierungseinrichtung zumindest eine oder eine Mehrzahl von Leitungen zum Leiten eines Wärmeträger, wobei sich die Leitungen vorzugsweise zumindest teilweise durch den Aufnahmekörper erstrecken. Bei dem Wärmeträger handelt es sich beispielsweise um ein Gas, wie Luft, oder um einen flüssigen Wärmeträger, wie beispielsweise Wasser, Thermalöl, Glycol oder Salzlösungen oder Mineralöl. Die Leitung erstreckt sich beispielsweise unterhalb der Lagerplätze, insbesondere in einer Ebene, vorzugsweise spiralförmig oder wendelförmig. Vorzugsweise ist die Leitung zumindest teilweise um die Lagerplätze herum angeordnet.

Bei der Heiz-/Kühleinrichtung handelt es sich vorzugsweise um ein Aggregat, das zum Heizen und/ oder Kühlen eines des Wärmeträgers ausgebildet ist. Beispielsweise umfasst eine Heiz-/Kühleinrichtung einen Wärmetauscher oder ein elektrisches Heiz- oder Kühlaggregat. Die Temperierungseinrichtung umfasst insbesondere einen Pufferspeicher, um kurzfristige Temperaturschwankungen des Wärmeträgers abzupuffern. Des Weiteren weist die Temperierungseinrichtung beispielsweise ein Gebläse oder einen Verdichter und insbesondere weitere Rohrleitungen zum Verbinden der Komponenten der Temperierungseinrichtung auf.

Der Aufnahmekörper ist gemäß einer weiteren Ausführungsform aus einem Metall ausgebildet ist. Bei dem Metall handelt es sich vorzugsweise um Aluminium oder Stahl. Vorzugsweise weisen die in dem Aufnahmekörper ausgebildeten Lagerplätze eine Geometrie auf, die in etwa der Geometrie eines Probenbehälters entspricht, sodass dieser in dem Lagerplatz, vorzugsweise mit keinem oder nur sehr geringem Spiel, aufnehmbar ist. Ein aus Metall ausgebildeter Aufnahmekörper sorgt für eine optimale Wärmeleitfähigkeit, sodass die über die Leitungen des Wärmeträgers in das Probenlager eingebrachte Wärme oder Kälte optimal an die Lagerplätze und die darin gelagerten Probenbehälter weitergeleitet wird.

Gemäß einer weiteren Ausführungsform weist das Probenlager zumindest eine oder eine Mehrzahl von Leitungen zum Leiten einer Aktivierungsflüssigkeit auf, sodass die Temperatur der Aktivierungsflüssigkeit in den Leitungen mittels der Temperierungseinrichtung einstellbar ist. Die Leitungen zum Leiten der Aktivierungsflüssigkeit erstrecken sich vorzugsweise durch den Aufnahmekörper. Insbesondere sind die Leitungen zum Leiten der Aktivierungsflüssigkeit derart in dem Aufnahmekörper angeordnet, dass sie durch die Leitungen zum Leiten des Wärmeträgers temperiert, insbesondere gekühlt oder erwärmt werden. Die Leitungen zum Leiten der Aktivierungsflüssigkeit sind vorzugsweise direkt neben den Leitungen zum Leiten des Wärmeträgers angeordnet. Es ist ebenfalls denkbar, dass sich die Leitungen zum Leiten der Aktivierungsflüssigkeit vollständig oder teilweise außen entlang des Aufnahmekörpers erstrecken. Bei der Aktivierungsflüssigkeit handelt es sich beispielsweise um Wasser oder destilliertes Wasser, das zur Aktivierung des Probenmaterials verwendbar ist. Beispielsweise weist sind die Leitungen zum Leiten der Aktivierungsflüssigkeit mit einem Speicher, insbesondere Tank, zur Zwischenspeicherung von Aktivierungsflüssigkeit verbunden. Der Speicher ist beispielsweise innerhalb des Probenlagers angeordnet und mittels des in den Leitungen geführten Wärmeträgers temperierbar. Es ist ebenfalls denkbar, dass der Speicher außerhalb des Probenlagers angeordnet ist und über Leitungen mit diesem, insbesondere mit den Leitungen zum Leiten der Aktivierungsflüssigkeit, verbunden ist. Vorzugsweise ist die Verweilzeit der Aktivierungsflüssigkeit innerhalb des Probenlagers, insbesondere innerhalb der durch den oder entlang des Aufnahmekörpers verlaufenden Leitungen zum Leiten der Aktivierungsflüssigkeit einstellbar.

Gemäß einer weiteren Ausführungsform weist der Aufnahmekörper einen oberen Bereich und einen dazu separaten unteren Bereich auf, wobei die Leitungen zum Leiten des Wärmeträgers und/ oder die Leitungen zum Leiten der Aktivierungsflüssigkeit in dem unteren Bereich angeordnet sind. Der untere Bereich und der obere Bereich sind vorzugsweise lösbar miteinander verbunden, beispielsweise verschraubt. Die Leitungen zum Leiten der Aktivierungsflüssigkeit und die Leitungen zum Leiten des Wärmeträgers sind vorzugsweise ausschließlich im unteren Bereich angeordnet. Vorzugsweise ist der untere Bereich kastenförmig ausgebildet, sodass die Leitungen von oben in den unteren Bereich beispielsweise zur Wartung erreichbar sind. Die Lagerplätze sind vorzugsweise ausschließlich in dem oberen Bereich des Aufnahmekörpers angeordnet. Ein geteilter Aufnahmekörper vereinfacht die Wartung der Leitung zum Leiten des Wärmeträgers und der Aktivierungsflüssigkeit.

Gemäß einer weiteren Ausführungsform weist das Probenlager zumindest eine Temperaturmesseinrichtung zur Ermittlung der Temperatur des Aufnahmekörpers und/ oder der Lagerplätze auf. Vorzugsweise ist sind eine Mehrzahl von Temperaturmesseinrichtung an dem Aufnahmekörper angebracht und beispielsweise gleichmäßig zueinander beabstandet. Insbesondere ist die Temperaturmesseinrichtung derart ausgebildet, dass sie ein Temperaturprofil vorzugsweise über zumindest eine Messebene innerhalb des Aufnahmekörpers ermittelt.

Gemäß einer weiteren Ausführungsform weist das Probenlager eine Steuerungs-/Regelungseinrichtung auf, die derart ausgebildet ist, dass sie die Temperatur des Probenlagers in Abhängigkeit der mit der Temperaturmesseinrichtung ermittelten Temperatur steuert/ regelt. Unter Steuern/ Regen ist vorzugsweise Steuern und/ oder Regeln zu verstehen. Die Temperaturmesseinrichtung ist insbesondere mit der Steuerungs-/Regelungseinrichtung zur Übermittlung der ermittelten Temperatur verbunden.

Vorzugsweise wird die Temperatur des Probenlagers, insbesondere des Aufnahmekörpers, vorzugsweise der Lagerplätze mittels der Steuerungs-/Regelungseinrichtung eingestellt. Vorzugsweise ist in der Steuerungs-/Regelungseinrichtung ein Temperatursollwert hinterlegt. Die Steuerungs-/Regelungseinrichtung ist vorzugsweise derart ausgebildet, dass sie die ermittelte Temperatur des Probenlagers, insbesondere der Lagerplätze, mit dem Temperatursollwert vergleicht und bei einer Abweichung der ermittelten Temperatur von dem Temperatursollwert, die Temperatur des Probenlagers, insbesondere der Lagerplätze, erhöht oder verringert, sodass diese dem Temperatursollwert entspricht.

Die Steuerungs- /Regelungseinrichtung ist vorzugsweise mit der Temperierungseinrichtung zur Einstellung der Temperatur des Probenlagers verbunden. Zur Änderung der Temperatur des Probenlagers, insbesondere der Lagerplätze, wird die Temperatur des durch die Leitung strömenden Wärmeträgers eingestellt, insbesondere erhöht oder verringert. Vorzugsweise wird der Wärmeträger mittels einer Heiz- oder Kühleinrichtung, wie einem Wärmetauscher oder einem elektrischen Kühl- oder Heizaggregat, erwärmt oder gekühlt. Der Temperatursollwert des Probenlagers entspricht beispielsweise einem ermittelten Temperaturwert eines dem Probenlager nachgeschalteten und separat zu diesem angeordneten Kalorimeter, insbesondere eines isothermalen Kalorimeters und dort insbesondere der Temperatur in einem Messraum des Kalorimeters.

Gemäß einer weiteren Ausführungsform weist das Probenlager eine Steuerungs-/Regelungseinrichtung auf, die derart ausgebildet ist, dass sie die Verweilzeit der Probenbehälter in den Lagerplätzen in Abhängigkeit der ermittelten Temperatur steuert/ regelt. Vorzugsweise ist die Steuerungs- /Regelungseinrichtung derart ausgebildet, dass sie die Temperaturdifferenz zwischen dem Probenlager, insbesondere den Lagerplätzen, und dem Temperatursollwert oder der in dem Kalorimeter ermittelten Temperatur berechnet und die Verweilzeit des Probenbehälters in dem Probenlager in Abhängigkeit der berechneten Temperaturdifferent steuert/ regelt. Vorzugsweise entspricht die ermittelte Verweilzeit, insbesondere zusammen mit den Materialkennwerten, wie Feinheit und Masse, einer Verweilzeit des Probenbehälters innerhalb des Probenlagers. Die Verweilzeit wird vorzugsweise derart gewählt, dass sich die Temperatur des Probenbehälters und beispielsweise seines Inhalts der Temperatur des Aufnahmekörpers, insbesondere der Lagerplätze, über die Verweilzeit angleicht.

Gemäß einer weiteten Ausführungsform sind die Lagerplätze als Aussparungen in dem Aufnahmekörper ausgebildet, sodass die Probenbehälter zumindest teilweise innerhalb des Aufnahmekörpers anordbar sind. Die Aussparungen sind beispielsweise Bohrungen oder Vertiefungen in dem Aufnahmekörper und sind insbesondere gleichmäßig zueinander beabstandet und vorzugsweise in mehreren Reihen angeordnet. Vorzugsweise sind die Aussparungen derart ausgebildet, dass der Probebehälter in einer Aussparung fixierbar ist. Die Tiefe der Aussparungen entsprechen vorzugsweise mindestens 5% der Höhe eines Probenbehälters, insbesondere 4 Mal der Höhe eines Probenbehälters, vorzugsweise 2 Mal der Höhe eines Probenbehälters, insbesondere 0,5 bis 0,9 Mal der Höhe des Probenbehälters.

Die Dosiereinrichtung, das Probenlager, die Mischvorrichtung und das Kalorimeter sind vorzugsweise separate Bauteile. Insbesondere wird die Materialprobe in dem Probenbehälter manuell oder automatisiert in dem Probenlager gelagert, von dem Probenlager in die Mischvorrichtung und von der Mischvorrichtung in das Kalorimeter verbracht.

Die Dosiereinrichtung umfasst beispielsweise eine Waage zum Ermitteln des Gewichts des Probematerials. Die Dosiereinrichtung ist vorzugsweise manuell oder automatisch bedienbar. Vorzugsweise umfasst die Dosiereinrichtung ein Förderelement zum Fördern des Probenmaterial, beispielsweise pneumatisch oder mechanisch.

Die Mischvorrichtung dient vorzugsweise zur Vorbereitung des Probematerials auf eine kalorimetrische Messung und weist beispielsweise eine Aufnahmeeinrichtung zur Aufnahme des Probenbehälters auf. Bei der Aufnahmeeinrichtung handelt es sich beispielsweise um eine Kapsel, eine Mischkammer oder eine Klemmvorrichtung mittels welcher der mit dem Probematerial gefüllte Probenbehälter fixierbar ist. Die Mischvorrichtung weist des Weiteren einen Rahmen auf. An der Aufnahmeeinrichtung der Mischvorrichtung sind vorzugsweise Vibratoren angebracht, die vorzugsweise pneumatisch angetrieben werden und den Probenbehälter in eine vertikale und/ oder horizontale Schwingung versetzen können. Die Aufnahmeeinrichtung der Mischvorrichtung ist insbesondere über Schwingungsdämpfer mit dem Rahmen verbunden, die der Befestigung der Aufnahmeeinrichtung an dem Rahmen dienen, sodass die Schwingung der Aufnahmeeinrichtung kaum oder gar nicht auf den Rahmen übertragen wird.

Ein Kalorimeter, insbesondere ein isothermes Wärmeflusskalorimeter. Ist derart ausgebildet, dass es die von dem Probewerkstoff abgegebene Reaktionswärme ermittelt. Die freigegebene Reaktionswärme und der Verlauf der Wärmeabgabe über die Zeit sind charakteristisch für die Reaktivität eines Probewerkstoffs, insbesondere eines Bindemittels. Die Zugabe einer Aktivierungsflüssigkeit, wie beispielsweise Wasser, zu den Werkstoffkomponenten startet den Hydratationsprozess, wobei die in den Werkstoffkomponenten gespeicherte Energie in Form von Reaktionswärme freigesetzt wird. Die kalorimetrische Messeinrichtung ermöglicht eine einfache und schnelle Ermittlung der Reaktivität des Probewerkstoffs.

Gemäß einer weiteren Ausführungsform ist eine weitere Dosiereinrichtung zum Dosieren einer Aktivierungsflüssigkeit in den in der Mischvorrichtung aufgenommenen Probenbehälter vorgesehen ist. Vorzugsweise wird die Aktivierungsflüssigkeit in den Probenbehälter dosiert bevor dieser in der Mischvorrichtung gemischt wird. Zur Dosierung der Aktivierungsflüssigkeit ist der Probenbehälter beispielsweise in der Mischvorrichtung aufgenommen und insbesondere geöffnet, sodass die Dosiereinrichtung die Aktivierungsflüssigkeit in den Probenbehälter ein dosieren kann. Vorzugsweise wird die Temperatur der Aktivierungsflüssigkeit vor der Dosierung in den Probenbehälter eingestellt. Insbesondere ist die weitere Dosiereinrichtung zur Einstellung der Temperatur der Aktivierungseinrichtung ausgebildet.

Gemäß einer weiteren Ausführungsform weist das Kalorimeter eine Temperaturmesseinrichtung zur Ermittlung der Temperatur des Kalorimeters auf und wobei die Temperaturmesseinrichtung zur Übermittlung der ermittelten Temperatur mit der Steuerungs-/Regelungseinrichtung verbunden ist. Das Kalorimeter weist vorzugsweise einen Messraum auf, in welchem die kalorimetrische Messung erfolgt. Die Temperaturmesseinrichtung ist vorzugsweise in dem Messraum angeordnet. Der ermittelte Temperaturwert stellt vorzugsweise einen Temperatursollwert zur Einstellung der Temperatur des Probenlagers, insbesondere der Lagerplätze dar.

Zur Steuerung/ Regelung der Temperatur des Kalorimeters, insbesondere des Messraums des Kalorimeters, ist die Steuerungs- /Regelungseinrichtung vorzugsweise derart ausgebildet, dass sie die ermittelten Temperaturdaten des Kalorimeters, insbesondere des Messraums des Kalorimeters, mit einem vorabbestimmten Temperatursollwert vergleicht und bei einer Abweichung der ermittelten Temperatur von dem Temperatursollwert, die Temperatur des Kalorimeters, insbesondere des Messraums des Kalorimeters, erhöht oder verringert, sodass diese dem Temperatursollwert entspricht.

Die Erfindung umfasst auch ein Verfahren zum Ermitteln der Reaktivität eines Probematerials aufweisend die Schritte:
Dosieren des Probematerials in einen Probenbehälter,
Temperieren des Probenbehälters in einem Probenlager, wie vorangehend beschrieben,
beispielsweise Temperieren einer Aktivierungsflüssigkeit
Zuführen des Probenbehälters in eine Mischvorrichtung zum Mischen des Probenmaterials in dem Probenbehälter, und
Ermitteln der Reaktivität des Probematerials in dem Behälter.

Die mit Bezug auf die Anlage zur Bestimmung der Reaktivität eines Probenmaterials beschriebenen Ausführungen und Vorteile treffen in verfahrensgemäßer Entsprechung ebenfalls auf das Verfahren zur Bestimmung der Reaktivität eines Probenmaterials zu.

Gemäß einer weiteren Ausführungsform wird die Temperatur innerhalb des Kalorimeters, und/ oder die Temperatur des Aufnahmekörpers ermittelt, wobei die Temperatur des Aufnahmekörpers des Probenlagers in Abhängigkeit der ermittelten Temperatur gesteuert/ geregelt.

Gemäß einer weiteren Ausführungsform wird die Verweilzeit des Probenbehälters in dem Probenlager vorzugsweise vor dem Beginn der kalorimetrischen Messung in Abhängigkeit der ermittelten Temperatur gesteuert/ geregelt.

Gemäß einer weiteren Ausführungsform erfolgt in dem Probenlager das Temperieren einer Aktivierungsflüssigkeit, wobei die Aktivierungsflüssigkeit anschließend in den Probenbehälter dosiert wird. Vorzugsweise wird die Verweilzeit der Aktivierungsflüssigkeit in dem Probenlager vorzugsweise vor dem Beginn der kalorimetrischen Messung in Abhängigkeit der ermittelten Temperatur gesteuert/ geregelt.

### Beschreibung der Zeichnungen

Die Erfindung ist nachfolgend anhand mehrerer Ausführungsbeispiele mit Bezug auf die beiliegenden Figuren näher erläutert.
- Fig. 1: zeigt eine schematische Darstellung eines Probenlagers in einer perspektivischen Ansicht gemäß einem Ausführungsbeispiel.
- Fig. 2: zeigt eine schematische Darstellung eines Probenlagers in einer Draufsicht auf den unteren Bereich des Aufnahmekörpers gemäß einem weiteren Ausführungsbeispiel.
- Fig. 3: zeigt eine schematische Darstellung eines Probenlagers in einer Querschnittsansicht gemäß einem weiteren Ausführungsbeispiel.
- Fig. 4: zeigt eine schematische Darstellung einer Anlage zur Bestimmung der Reaktivität eines Probenmaterials gemäß einem weiteren Ausführungsbeispiel.

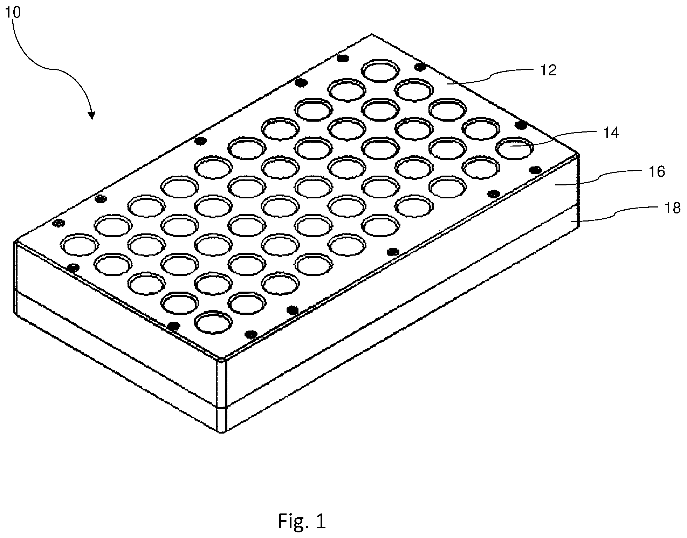

Fig. 1 zeigt ein Probenlager 10 zur Aufnahme einer Mehrzahl von in Fig. 1 nicht dargestellten Probenbehältern, in denen Probenmaterial gelagert ist. Das Probematerial ist beispielsweise ein Klinker oder ein hydraulisches Bindemittel mit unterschiedlichen Zusammensetzungen verschiedener Werkstoffkomponenten, wie beispielsweise Klinker, Sulfatträger oder Zumahlstoffe aufweisen. Zumahlstoffe sind beispielsweise Hüttensand, Flugasche, Puzzolan, Kalkstein oder kalzinierter Ton. Das Probematerial ist vorzugsweise gemahlen, insbesondere pulverförmig.

Das Probenlager 10 weist einen Aufnahmekörper 12 auf, der eine Mehrzahl von Aufnahmemitteln, insbesondere Lagerplätzen 14 aufweist. Jeder Lagerplatz 14 ist vorzugsweise zur Aufnahme jeweils genau eines Probenbehälters ausgebildet. Bei dem Probenbehälter handelt es sich beispielsweise um eine Ampulle, die vorzugsweise mit einem Deckel verschließbar ist. Der Deckel ist beispielsweise um ein Gewinde gemäß einem Drehverschluss auf die Ampulle aufschraubbar. Der Probenbehälter weist vorzugsweise einen runden, insbesondere kreisförmigen Querschnitt auf. Vorzugsweise ist der Probenbehälter aus HD-PE (high density Polyethylen), PET, Polycarbonat, Polypropylen oder Polystyrol ausgebildet.

Der Aufnahmekörper 12 ist vorzugsweise aus einem Metall, wie beispielsweise Aluminium oder Stahl. ausgebildet. Insbesondere ist der Aufnahmekörper 12 kastenförmig ausgebildet. Beispielhaft ist der Aufnahmekörper 12 der Fig. 1 zweiteilig ausgebildet und weist einen oberen Bereich 16 und einen unteren Bereich 18 auf. Es ist ebenfalls denkbar, dass der Aufnahmekörper 12 einteilig oder mehrteilig mit mehr als zwei Teilen ausgebildet ist.

Der obere Bereich 16 des Aufnahmekörpers 12 ist beispielsweise kastenförmig oder als Vollblock ausgebildet und weist die Lagerplätze 14 auf. Die Lagerplätze 14 sind beispielsweise als im Wesentlichen vertikale Aussparungen, insbesondere Bohrungen von oben in den Aufnahmekörper 12, vorzugsweise den oberen Bereich 16 des Aufnahmekörpers 12 ausgebildet. Die Lagerplätze 14 sind beispielsweise zylinderförmig ausgebildet und weisen einen runden, insbesondere kreisförmigen konstanten Querschnitt auf. Die Tiefe der Lagerplätze 14 entspricht vorzugsweise mindestens 5% der Höhe des darin aufzunehmenden Probenbehälters, vorzugsweise maximal vier Mal der Höhe des Probenbehälters, insbesondere 0,9 -2, insbesondere 0,5 Mal der Höhe des Probenbehälters.

Beispielhaft weist das Probenlager 10 fünfzig Lagerplätze auf, die in Reihen nebeneinander angeordnet und vorzugsweise gleichmäßig zueinander beabstandet sind. Insbesondere ist die Anzahl der Lagerplätze zehn bis hundert, vorzugsweise zwanzig bis achtzig, insbesondere fünfzig.

Der untere Bereich 18 des Aufnahmekörpers 12 ist beispielsweise kastenförmig oder als Vollblock ausgebildet und weist vorzugsweise zumindest eine Leitung auf, insbesondere eine Mehrzahl von Leitungen zum Leiten eines Wärmeträgers und/ oder einer Aktivierungsflüssigkeit. Bei dem Wärmeträger handelt es sich beispielsweise um ein Gas, wie Luft, oder um einen flüssigen Wärmeträger, wie beispielsweise Wasser, Thermalöl, Glykol oder Salzlösungen oder Mineralöl. Der obere Bereich 16 und der untere Bereich 18 des Aufnahmekörpers 12 sind vorzugsweise miteinander über ein Verbindungsmittel, wie Schrauben, verbunden oder beispielsweise stoffschlüssig miteinander verbunden, beispielsweise verschweißt. Insbesondere sind der obere Bereich 16 und der untere Bereich 18 des Aufnahmekörpers 12 lösbar miteinander verbunden.

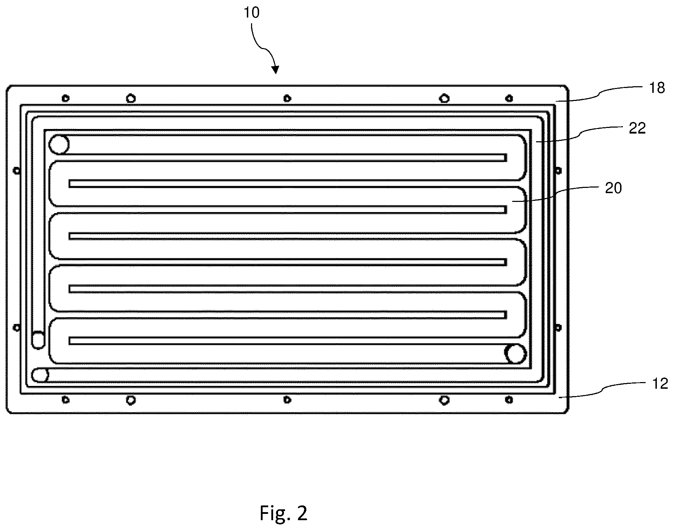

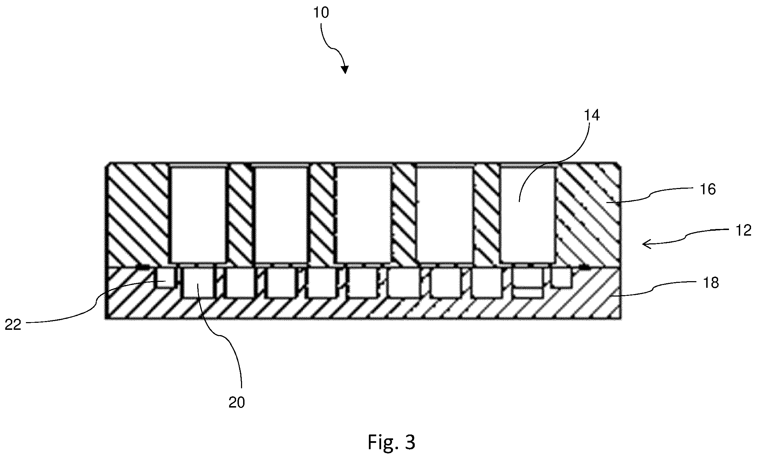

Fig. 2 zeigt eine Ansicht eines Probenlagers 10 Draufsicht auf den unteren Bereich des Aufnahmekörpers 12 und Fig. 3 zeigt eine Querschnittsansicht des Probenlagers. Das Probenlager 10 weist beispielhaft eine Leitung 20 zum Leiten eines Wärmeträgers auf. Bei dem Wärmeträger handelt es sich beispielsweise um ein Gas, wie Luft, oder um einen flüssigen Wärmeträger, wie beispielsweise Wasser, Thermalöl, Glykol oder Salzlösungen oder Mineralöl. Die Leitung 20 ist vorzugsweise Teil einer Temperierungseinrichtung, die zusätzlich zu der Leitung 20 einen nicht dargestellten Wärmeträgerkreislauf umfasst. Die Temperierungseinrichtung weist vorzugsweise einen Wärmetauscher oder Heiz-/Kühlaggregat zum Temperieren des Wärmeträgers auf. Des Weitere weist die Temperierungseinrichtung ein Gebläse oder einen Verdichter und insbesondere weitere Rohrleitungen zum Verbinden der Komponenten auf.

Die Leitung 20 ist vorzugsweise unterhalb der Lagerplätze 14 angeordnet. Es ist ebenfalls denkbar, dass die Leitung 20 zumindest teilweise die Lagerplätze 14, insbesondere in dessen unteren Bereichen, umschließt. Vorzugsweise ist die Leitung 20 wendelförmig ausgebildet. Insbesondere ist die Leitung 20 in einer Ebene angeordnet und erstreckt sich derart über die Ebene, beispielsweise spiralförmig oder über eine Mehrzahl von Windungen.

Das Probenlager 10 weist beispielhaft eine weitere Leitung 22 zum Leiten einer Aktivierungsflüssigkeit, wie beispielsweise Wasser oder destilliertes Wasser, auf. Die Leitung 22 zum Leiten einer Aktivierungsflüssigkeit ist insbesondere außen um die Leitung 20 zum Leiten des Wärmeträgers herum angeordnet und erstreckt sich beispielhaft innerhalb des untere Bereichs 16 des Aufnahmekörpers 12 von innen entlang der Seitenflächen des unteren Bereichs 16 des Aufnahmekörpers 12. Die Leitung 22 zum Leiten einer Aktivierungsflüssigkeit ist beispielsweise Teil eines nicht dargestellten Kreislaufs der Aktivierungsflüssigkeit, wobei der Kreislauf vorzugsweise einen Tank zum Speichern einer Aktivierungsflüssigkeit und weitere Leitungen zum Verbinden des Tanks mit dem Probenlager 10 umfasst. Die Leitung 22 zum Leiten einer Aktivierungsflüssigkeit weist beispielhaft einen geringeren Durchmesser auf als die Leitung 20 zum Leiten des Wärmeträgers.

Die Leitung 20 zum Leiten des Wärmeträgers dient der Temperierung der Probenbehälter in den jeweiligen Lagerplätzen 14. Vorzugsweise ist die Temperatur des Wärmeträgers einstellbar. Die Leitung 22 zum Leiten einer Aktivierungsflüssigkeit dient der Temperierung der Aktivierungsflüssigkeit, sodass diese vorzugsweise die gleiche Temperatur aufweist wie die Probenbehälter, insbesondere das Probenmaterial innerhalb der Probenbehälter.

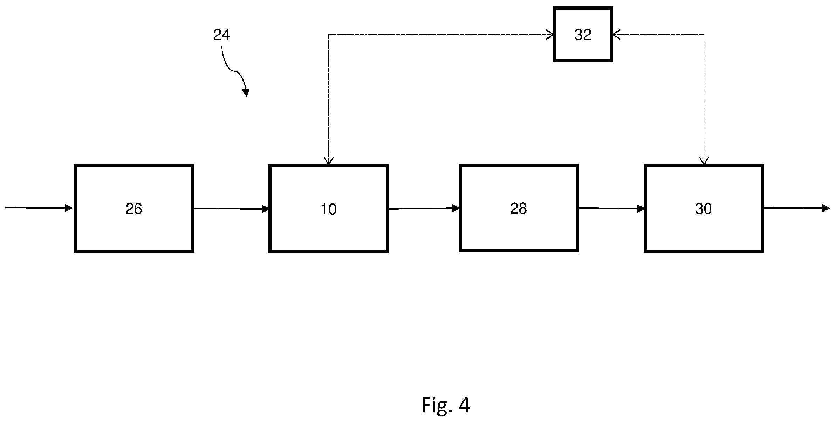

Fig. 4 zeigt eine schematische Darstellung einer Anlage zur Bestimmung der Reaktivität eines Probenmaterial. Die Anlage 24 umfasst eine Dosierungseinrichtung 26 zum Dosieren von Probenmaterial in einen Probenbehälter. Die Dosiereinrichtung 26 umfasst beispielweise eine Waage zur Bestimmung des Probengewichts und zur Dosierung eines vorabbestimmten Gewichts an Probenmaterial in den Probenbehälter. Die Anlage 24 umfasst des Weiteren ein Probenlager, wie vorangehend mit Bezug auf die Fig. 1 bis 3 beschrieben. Der in der Dosiereinrichtung 26 mit dem Probenmaterial befüllte Probenbehälter wird in das Probenlager 10 manuell oder automatisch zugeführt und vorzugsweis in einem Lagerplatz 14 des Probenlagers 10 aufgenommen.

Die Anlage 24 umfasst des Weiteren vorzugsweise eine Mischvorrichtung 28 zum Mischen des Probenmaterials in dem Probenbehälter. Vorzugsweise weist die Mischvorrichtung 28 eine weitere Dosiereinrichtung zum Dosieren einer Aktivierungsflüssigkeit in den Probenbehälter auf. Dazu wird der Probenbehälter vorzugsweise geöffnet, insbesondere aufgeschraubt, und die Aktivierungsflüssigkeit wird in den Probenbehälter injiziert. Anschließend wird der Probenbehälter vorzugsweise wieder verschlossen und das Probematerial wird mit der Aktivierungsflüssigkeit in der Mischvorrichtung 28 vermischt.

Die Mischvorrichtung 28 dient vorzugsweise zur Vorbereitung des Probematerials auf eine kalorimetrische Messung insbesondere der Herstellung Paste aus einer Probe und einer Aktivierungsflüssigkeit. Bei einer kalorimetrischen Messung handelt es sich insbesondere um eine Ermittlung der von dem Probematerial abgegebenen Wärme nach Zugabe einer Anregerflüssigkeit, wie beispielsweise Wasser oder destilliertes Wasser. zu dem Probematerial. Die abgegebene Wärme ist ein Maß für die in dem Probematerial gespeicherte Energie und die Freisetzung dieser Energie über der Zeit.

Die Mischvorrichtung 28 weist beispielsweise eine Aufnahmeeinrichtung zur Aufnahme des Probenbehälters auf. Bei der Aufnahmeeinrichtung handelt es sich beispielsweise um eine Kapsel, eine Mischkammer oder eine Klemmvorrichtung mittels welcher der mit dem Probematerial gefüllte Probenbehälter fixierbar ist. Die Mischvorrichtung 28 weist des Weiteren einen Rahmen auf. An der Aufnahmeeinrichtung der Mischvorrichtung sind vorzugsweise Vibratoren angebracht, die vorzugsweise pneumatisch angetrieben werden und den Probenbehälter in eine vertikale und/ oder horizontale Schwingung versetzen können. Die Aufnahmeeirichtung der Mischvorrichtung 28 ist insbesondere über Schwingungsdämpfer mit dem Rahmen verbunden, die der Befestigung der Aufnahmeeinrichtung an dem Rahmen dienen, sodass die Schwingung der Aufnahmeeinrichtung kaum oder gar nicht auf den Rahmen übertragen wird.

Die in dem Probenlager 10 zwischengelagerten und darin temperierten Probenbehälter werden der Mischvorrichtung 28 zugeführt. Vorzugsweise werden die Probenbehälter erst dann der Mischvorrichtung 28 zugeführt, wenn sie eine bestimmte vorabbestimmte Solltemperatur aufweisen.

Die Anlage 24 weist des Weiteren ein Kalorimeter 30 auf, insbesondere ein isothermes Wärmeflusskalorimeter, das der Bestimmung der Reaktivität des Probenmaterials dient. Die in der Mischvorrichtung 28 gemischten Probenbehälter werden vorzugsweise einzeln oder mehrere Probenbehälter gleichzeitig individuellen Messräumen in dem Kalorimeter 30 zugeführt.

Beispielhaft weist die Anlage 24 des Weiteren eine Steuerungs- /Regelungseinrichtung 32 auf. Es ist ebenfalls denkbar, dass die Steuerungs- /Regelungseinrichtung 32 Teil des Probenlagers 10 ist. Die Steuerungs- /Regelungseinrichtung 32 steht vorzugsweise mit dem Kalorimeter 30 und dem Probenlager 10 derart in Verbindung, dass sie die Temperatur des Probenlagers 10 und/ oder die Temperatur des Kalorimeters 30 steuert/ regelt. Vorzugsweise wird die Temperatur der Lagerplätze 14 und/ oder die Temperatur eines Messraums des Kalorimeters 30 mittel der Steuerungs- /Regelungseinrichtung 32 eingestellt. Vorzugsweise weist das Probenlager 10 und/ oder das Kalorimeter 30 jeweils eine Temperaturmesseinrichtung auf. Die in dem Probenlager 10 angebrachte Temperaturmesseinrichtung ist insbesondere zur Ermittlung der Temperatur zumindest eines oder mehrerer Lagerplätze 14 ausgebildet und angeordnet. Die in der Kalorimeter 30 angeordnete Temperaturmesseinrichtung ist vorzugsweise zur Ermittlung der Temperatur des Messraums des Kalorimeters ausgebildet und angeordnet. Die Temperaturmesseinrichtungen sind insbesondere mit der Steuerungs-/Regelungseinrichtung 32 zur Übermittlung der ermittelten Temperaturdaten verbunden.

Vorzugsweise ist in der Steuerungs- /Regelungseinrichtung 32 ein Temperatursollwert hinterlegt. Die Steuerungs- /Regelungseinrichtung 32 ist vorzugsweise derart ausgebildet, dass sie die ermittelten Temperaturdaten des Probenlagers 10, insbesondere der Lagerplätze 14, mit dem Temperatursollwert vergleicht und bei einer Abweichung der ermittelten Temperatur von dem Temperatursollwert, die Temperatur des Probenlagers 10, insbesondere der Lagerplätze 14, erhöht oder verringert, sodass diese dem Temperatursollwert entspricht.

Die Steuerungs- /Regelungseinrichtung 32 ist vorzugsweise mit der Temperierungseinrichtung zur Einstellung der Temperatur des Probenlagers 10 verbunden. Zur Änderung der Temperatur des Probenlagers 10, insbesondere der Lagerplätze 14, wird die Temperatur des durch die Leitung 20 strömenden Wärmeträgers eingestellt, insbesondere erhöht oder verringert. Vorzugsweise wird der Wärmeträger mittels einer Heiz- oder Kühleinrichtung, wie einem Wärmetauscher oder einem elektrischen Kühl- oder Heizaggregat, erwärmt oder gekühlt. Der Temperatursollwert des Probenlagers 10 entspricht beispielsweise dem ermittelten Temperaturwert des Kalorimeters 30, insbesondere der Temperatur in dem Messraum des Kalorimeters 30.

Zur Steuerung/ Regelung der Temperatur des Kalorimeters 30, insbesondere des Messraums des Kalorimeters 30, ist die Steuerungs- /Regelungseinrichtung 32 vorzugsweise derart ausgebildet, dass sie die ermittelten Temperaturdaten des Kalorimeters 30, insbesondere des Messraums des Kalorimeters 30, mit dem Temperatursollwert vergleicht und bei einer Abweichung der ermittelten Temperatur von dem Temperatursollwert, die Temperatur des Kalorimeters 30, insbesondere des Messraums des Kalorimeters 30, erhöht oder verringert, sodass diese dem Temperatursollwert entspricht.

Die Steuerungs- /Regelungseinrichtung 32 ist vorzugsweise derart ausgebildet, dass sie die Verweilzeit der Probenbehälter in dem Probenlager in Anhängigkeit der ermittelten Temperatur den Probenlagers 10, insbesondere der Lagerplätze 14 steuert/ regelt. Vorzugsweise ist die Steuerungs- /Regelungseinrichtung 32 derart ausgebildet, dass sie die Temperaturdifferenz zwischen dem Probenlager 10, insbesondere den Lagerplätzen 14, und dem Temperatursollwert oder der in dem Kalorimeter 30 ermittelten Temperatur berechnet und die Verweilzeit des Probenbehälters in dem Probenlager 10 in Abhängigkeit der berechneten Temperaturdifferent steuert/ regelt.

### Bezugszeichenliste

- 10: Probenlager
- 12: Aufnahmekörper
- 14: Lagerplätze
- 16: oberer Bereich des Aufnahmekörpers
- 18: unterer Bereich des Aufnahmekörpers
- 20: Leitung zum Leiten eines Wärmeträgers
- 22: Leitung zum Leiten einer Aktivierungsflüssigkeit
- 24: Anlage zur Bestimmung der Reaktivität eines Probenmaterial
- 26: Dosierungseinrichtung
- 28: Mischvorrichtung
- 30: Kalorimeter
- 32: Steuerungs- /Regelungseinrichtung

## Patentansprüche

1. Anlage (24) zur Bestimmung der Reaktivität eines Probenmaterials aufweisend:
eine Dosiereinrichtung (26) zum Dosieren des Probenmaterials in einen Probenbehälter, ein Probenlager (10) zum Temperieren des Probematerials in dem Probenbehälter, eine Mischvorrichtung (28) zur Aufnahme des Probenbehälters mit dem temperierten Probematerial und zum Mischen des Probenmaterials in dem Probenbehälter und ein Kalorimeter (30) zur Ermittlung der Reaktivität des gemischten Probenmaterials, wobei das Probenlager (10) zur Vorbereitung eines Probematerials der Zement- oder Kalkindustrie auf eine kalorimetrische Messung, aufweist
einen Aufnahmekörper (12) mit
zumindest einem oder eine Mehrzahl von Lagerplätzen (14) zur Aufnahme jeweils eines Probenbehälters mit einem Probematerial,
**dadurch gekennzeichnet, dass**
das Probenlager (10) eine Temperierungseinrichtung zum Kühlen oder Erwärmen der Lagerplätze (14) des Aufnahmekörpers (12) aufweist.

2. Anlage (24) nach Anspruch 1, wobei die Temperierungseinrichtung Leitungen (20) zum Leiten des Wärmeträgers und eine Heiz-/Kühleinrichtung umfasst.

3. Anlage (24) nach einem der vorangehenden Ansprüche, wobei der Aufnahmekörper (12) aus einem Metall ausgebildet ist.

4. Anlage (24) nach einem der vorangehenden Ansprüche, wobei das Probenlager (10) Leitungen (22) zum Leiten einer Aktivierungsflüssigkeit aufweist, sodass die Temperatur der Aktivierungsflüssigkeit in den Leitungen (22) mittels der Temperierungseinrichtung einstellbar ist.

5. Anlage (24) nach Anspruch 2 und 4, wobei der Aufnahmekörper (12) einen oberen Bereich (16) und einen dazu separaten unteren Bereich (18) aufweist und wobei die Leitungen (20) zum Leiten des Wärmeträgers und/ oder die Leitungen (22) zum Leiten der Aktivierungsflüssigkeit in dem unteren Bereich (18) angeordnet sind.

6. Anlage (24) nach einem der vorangehenden Ansprüche, wobei das Probenlager (10) zumindest eine Temperaturmesseinrichtung zur Ermittlung der Temperatur des Aufnahmekörpers (12) und/ oder der Lagerplätze (14) aufweist.

7. Anlage (24) nach Anspruch 6, wobei das Probenlager (10) eine Steuerungs-/Regelungseinrichtung (32) aufweist, die derart ausgebildet ist, dass sie die Temperatur des Probenlagers (10) in Abhängigkeit der mit der Temperaturmesseinrichtung ermittelten Temperatur steuert/ regelt.

8. Anlage (24) nach Anspruch 6, wobei das Probenlager (10) eine Steuerungs-/Regelungseinrichtung (32) aufweist, die derart ausgebildet ist, dass sie die Verweilzeit der Probenbehälter in den Lagerplätzen (14) in Abhängigkeit der mit der Temperaturmesseinrichtung ermittelten Temperatur steuert/ regelt.

9. Anlage (24) nach einem der vorangehenden Ansprüche, wobei die Lagerplätze (14) als Aussparungen in dem Aufnahmekörper (12) ausgebildet sind, sodass die Probenbehälter zumindest teilweise innerhalb des Aufnahmekörpers (12) anordbar sind.

10. Anlage (24) nach einem der vorangehenden Ansprüche, wobei eine weitere Dosiereinrichtung zum Dosieren einer Aktivierungsflüssigkeit in den in der Mischvorrichtung (28) aufgenommenen Probenbehälter vorgesehen ist.

11. Anlage (24) nach einem der vorangehenden Ansprüche, wobei das Kalorimeter (30) eine Temperaturmesseinrichtung zur Ermittlung der Temperatur des Kalorimeters (30) aufweist und wobei die Temperaturmesseinrichtung zur Übermittlung der ermittelten Temperatur mit der Steuerungs-/Regelungseinrichtung (32) verbunden ist.

12. Verfahren zum Ermitteln der Reaktivität eines Probematerials mit einer Anlage (24) nach einem der vorangehenden Ansprüche aufweisend die Schritte:
Dosieren des Probematerials in einen Probenbehälter,
Temperieren des Probenbehälters in einem Probenlager (10),
Zuführen des Probenbehälters in eine Mischvorrichtung (28) zum Mischen des Probenmaterials in dem Probenbehälter, und
Ermitteln der Reaktivität des Probematerials in dem Probenbehälter mittels eines Kalorimeters (30).

13. Verfahren nach Anspruch 12, wobei die Temperatur innerhalb des Kalorimeters (30), und/ oder die Temperatur des Aufnahmekörpers (12) ermittelt wird und wobei die Temperatur des Aufnahmekörpers (12) des Probenlagers (10) in Abhängigkeit der ermittelten Temperatur gesteuert/ geregelt wird.

14. Verfahren nach einem der Ansprüche 12 bis 13, wobei die Verweilzeit des Probenbehälters in dem Probenlager (10) in Abhängigkeit der ermittelten Temperatur gesteuert/ geregelt wird.

15. Verfahren nach einem der Ansprüche 12 bis 14, wobei in dem Probenlager (10) das Temperieren einer Anregerflüssigkeit erfolgt und wobei die Anregerflüssigkeit anschließend in den Probenbehälter dosiert wird.

## Claims

1. A system (24) for determining the reactivity of a sample material, having: a metering device (26) for metering the sample material into a sample vessel, a sample store (10) for adjusting the temperature of the sample material in the sample vessel, a mixing apparatus (28) for accommodating the sample vessel containing the sample material at controlled temperature and for mixing the sample material in the sample vessel, and a calorimeter (30) for ascertaining the reactivity of the mixed sample material, wherein the sample store (10), for preparation of a sample material in the cement or lime industry for calorimetric measurement, has the following:
an accommodation body (12) having
at least one or a multitude of storage sites (14) each for accommodation of one sample vessel containing a sample material,
**characterized in that**
the sample store (10) has a temperature control device for cooling or heating the storage sites (14) of the accommodation body (12).

2. The system (24) as claimed in claim 1, wherein the temperature control device comprises conduits (20) for conducting the heat transfer medium and a heating/cooling device.

3. The system (24) as claimed in either of the preceding claims, wherein the accommodation body (12) is formed from a metal.

4. The system (24) as claimed in any of the preceding claims, wherein the sample store (10) has conduits (22) for conducting an activation liquid, such that the temperature of the activation liquid in the conduits (22) is adjustable by means of the temperature control device.

5. The system (24) as claimed in claims 2 and 4, wherein the accommodation body (12) has an upper region (16) and a separate lower region (18) and wherein the conduits (20) for conducting the heat transfer medium and/or the conduits (22) for conducting the activation liquid are disposed in the lower region (18).

6. The system (24) as claimed in any of the preceding claims, wherein the sample store (10) has at least one temperature measurement device for ascertaining the temperature of the accommodation body (12) and/or the storage sites (14).

7. The system (24) as claimed in claim 6, wherein the sample store (10) has an open-loop/closed-loop control device (32) designed to control the temperature of the sample store (10) by open-loop/closed-loop control depending on the temperature ascertained with the temperature measurement device.

8. The system (24) as claimed in claim 6, wherein the sample store (10) has an open-loop/closed-loop control device (32) designed to control the dwell time of the sample vessel in the storage sites (14) by open-loop/closed-loop control depending on the temperature ascertained with the temperature measurement device.

9. The system (24) as claimed in any of the preceding claims, wherein the storage sites (14) take the form of recesses in the accommodation body (12), such that the sample vessels can be arranged at least partly within the accommodation body (12).

10. The system (24) as claimed in any of the preceding claims, wherein a further metering device is provided for metering an activation liquid into the sample vessel accommodated in the mixing apparatus (28).

11. The system (24) as claimed in any of the preceding claims, wherein the calorimeter (30) has a temperature measurement device for ascertaining the temperature of the calorimeter (30) and wherein the temperature measurement device is connected to the open-loop/closed-loop control device (32) for transmission of the temperature ascertained.

12. A method of ascertaining the reactivity of a sample material with a system (24) as claimed in any of the preceding claims, comprising the following steps:
metering the sample material into a sample vessel,
adjusting the temperature of the sample vessel in a sample store (10),
feeding the sample vessel into a mixing apparatus (28) for mixing the sample material in the sample vessel, and
ascertaining the reactivity of the sample material in the sample vessel by means of a calorimeter (30).

13. The method as claimed in claim 12, wherein the temperature within the calorimeter (30), and/or the temperature of the accommodation body (12) is ascertained and wherein the temperature of the accommodation body (12) of the sample store (10) is controlled by open-loop/closed-loop control depending on the temperature ascertained.

14. The method as claimed in either of claims 12 and 13, wherein the dwell time of the sample vessel in the sample store (10) is controlled by open-loop/closed-loop control depending on the temperature ascertained.

15. The method as claimed in any of claims 12 to 14, wherein the temperature of a trigger liquid is adjusted in the sample store (10) and wherein the trigger liquid is then metered into the sample vessel.

## Revendications

1. Système (24) pour déterminer la réactivité d'un matériau d'échantillonnage, comprenant : un dispositif de dosage (26) pour doser le matériau d'échantillonnage dans un récipient d'échantillonnage, un magasin d'échantillons (10) pour ajuster la température du matériau d'échantillonnage dans le récipient d'échantillonnage, un appareil de mélange (28) pour loger le récipient d'échantillonnage contenant le matériau d'échantillonnage à une température contrôlée et pour mélanger le matériau d'échantillonnage dans le récipient d'échantillonnage, et un calorimètre (30) pour vérifier la réactivité du matériau d'échantillonnage mélangé, dans lequel le magasin d'échantillons (10), pour la préparation d'un matériau d'échantillonnage dans l'industrie du ciment ou de la chaux en vue d'une mesure calorimétrique, présente les caractéristiques suivantes :
un corps de logement (12) comportant
au moins un ou une multitude de sites de stockage (14) pouvant accueillir chacun un récipient contenant un échantillon,
**caractérisé par le fait que**
le magasin d'échantillons (10) est doté d'un dispositif de régulation de la température pour refroidir ou chauffer les sites de stockage (14) du corps de logement (12).

2. Système (24) selon la revendication 1, dans lequel le dispositif de régulation de la température comprend des conduits (20) pour acheminer le fluide caloporteur et un dispositif de chauffage/refroidissement.

3. Système (24) selon l'une des revendications précédentes, dans lequel le corps de logement (12) est formé d'un métal.

4. Système (24) selon l'une quelconque des revendications précédentes, dans lequel la réserve d'échantillons (10) comporte des conduits (22) pour le passage d'un liquide d'activation, de sorte que la température du liquide d'activation dans les conduits (22) est réglable au moyen du dispositif de régulation de la température.

5. Le système (24) selon les revendications 2 et 4, dans lequel le corps de logement (12) a une région supérieure (16) et une région inférieure séparée (18) et dans lequel les conduits (20) pour conduire le fluide de transfert de chaleur et/ou les conduits (22) pour conduire le liquide d'activation sont disposés dans la région inférieure (18).

6. Le système (24) selon l'une des revendications précédentes, dans lequel le magasin d'échantillons (10) comporte au moins un dispositif de mesure de la température pour déterminer la température du corps d'hébergement (12) et/ou sites de stockage (14).

7. Système (24) selon la revendication 6, dans lequel la réserve d'échantillons (10) est équipée d'un dispositif de contrôle en boucle ouverte/fermée (32) conçu pour contrôler la température de la réserve d'échantillons (10) par un contrôle en boucle ouverte/fermée en fonction de la température déterminée par le dispositif de mesure de la température.

8. Système (24) selon la revendication 6, dans lequel le magasin d'échantillons (10) possède un dispositif de contrôle en boucle ouverte/fermée (32) conçu pour contrôler le temps de séjour du récipient d'échantillon dans les sites de stockage (14) par un contrôle en boucle ouverte/fermée en fonction de la température déterminée par le dispositif de mesure de la température.

9. Système (24) selon l'une quelconque des revendications précédentes, dans lequel les sites de stockage (14) se présentent sous la forme d'évidements dans le corps de logement (12), de sorte que les récipients d'échantillonnage peuvent être disposés au moins en partie à l'intérieur du corps de logement (12).

10. Le système (24) selon l'une quelconque des revendications précédentes, dans lequel un autre dispositif de dosage est prévu pour doser un liquide d'activation dans le récipient d'échantillonnage logé dans l'appareil de mélange (28).

11. Le système (24) selon l'une des revendications précédentes, dans lequel le calorimètre (30) possède un dispositif de mesure de la température pour déterminer la température du calorimètre (30) et dans lequel le dispositif de mesure de la température est connecté à au dispositif de contrôle en boucle ouverte/fermée (32) pour la transmission de la température déterminée.

12. Méthode de détermination de la réactivité d'un échantillon de matériau à l'aide d'un système (24) selon l'une quelconque des revendications précédentes, comprenant les étapes suivantes :
le dosage du matériau d'échantillonnage dans un récipient d'échantillonnage,
réglage de la température du récipient à échantillon dans un magasin d'échantillons (10),
introduire le récipient d'échantillonnage dans un appareil de mélange (28) pour mélanger le matériau d'échantillonnage dans le récipient d'échantillonnage, et
déterminer la réactivité de l'échantillon dans le récipient à l'aide d'un calorimètre (30).

13. Méthode selon la revendication 12, dans laquelle la température à l'intérieur du calorimètre (30) et/ou la température du corps d'hébergement (12) est déterminée et dans laquelle la température du corps d'hébergement (12) de l'entrepôt d'échantillons (10) est contrôlée par une commande en boucle ouverte/fermée en fonction de la température déterminée.

14. Méthode selon l'une des revendications 12 et 13, dans laquelle le temps de séjour du récipient d'échantillon dans le magasin d'échantillons (10) est contrôlé par une commande en boucle ouverte/fermée en fonction de la température déterminée.

15. Méthode selon l'une des revendications 12 à 14, dans laquelle la température d'un liquide de déclenchement est ajustée dans le magasin d'échantillons (10) et dans laquelle le liquide de déclenchement est ensuite dosé dans le récipient d'échantillonnage.
